# EUROPEAN PATENT APPLICATION

(11) **EP 4 560 646 A1**
(43) Date of publication of application: **28.05.2025**
(21) Application number: 23211391.0
(22) Date of filing: 22.11.2023
(51) Int. Cl.: G16H 40/63, G16H 30/40

(54) **METHOD FOR USE IN CONTRAST MEDICAL IMAGING**

(71) Applicant: Koninklijke Philips N.V., 5656 AG Eindhoven (NL)
(72) Inventor: BUELOW, Thomas, Eindhoven (NL); DESHPANDE, Hrishikesh Narayanrao, Eindhoven (NL); HARDER, Tim Philipp, 5656AG Eindhoven (NL); NORDHOFF, Tanja, Eindhoven (NL); SCHMITT, Holger, Eindhoven (NL)
(74) Representative: Philips Intellectual Property & Standards

(57) **Abstract**

The invention provides a computer-implemented method for use in contrast medical imaging, the method comprising generating input data representative of a plurality of patient parameters, wherein at least some of the input data is obtained from sensor data acquired by one or more sensors; inputting the input data into an AI model; and obtaining, using the AI model, operation parameters for operation of a contrast medical imaging system, the operation parameters comprising contrast injection parameters, and optionally comprising imaging parameters.

## Description

### FIELD OF THE INVENTION

The invention provides a computer-implemented method for use in contrast medical imaging, a medical imaging system, a computer program product, and a computer readable medium.

### BACKGROUND OF THE INVENTION

In the context of contrast medical systems, contrast injection parameters and imaging parameters have large impact on the imaging quality and on protection of the patient who is the subject of the imaging procedure. That is, the amount of contrast agent in the Region of Interest, ROI, of the imaging procedure at time of image capture impacts imaging quality, and particularly should not be too low. Moreover, for protecting the patient, as few images as possible should be taken and the amount of contrast agent and radiation dosage should be kept low. Accordingly, it is difficult to accommodate imaging quality and patient protection, particularly, considering that imaging procedures are carried out at different sessions, for different patients and/or different anatomies, which makes balancing the above requirements even more difficult.

It is an object of the invention to accommodate both, imaging quality and patient protection, in an improved manner.

### SUMMARY OF THE INVENTION

The invention provides a computer-implemented method for use in contrast medical imaging, a medical imaging system, a computer program product, and a computer readable medium according to the independent claims. Preferred embodiments are laid down in the dependent claims.

The present disclosure provides a computer-implemented method for use in contrast medical imaging. The method comprises generating input data representative of a plurality of patient parameters, wherein at least some of the input data is obtained from sensor data acquired by one or more sensors, inputting the input data into an artificial intelligence, AI, model, such as a machine learning, ML, model, and obtaining, using the AI model, operation parameters for operation of a contrast medical imaging system, the operation parameters comprising contrast injection parameters, and optionally comprising imaging parameters. In particular, at least some of the patient parameters may comprise or be derived from the sensor data.

In other words, the method comprises generating input data representative of a plurality of patient parameters, wherein at least some of the input data is obtained from sensor data acquired by one or more sensors, inputting the input data into an artificial intelligence, AI, model, such as a machine learning, ML, model, and obtaining, using the AI model, contrast injection parameters, and optionally comprising imaging parameters. The injection parameters are part of operation parameters for operation of a contrast medical imaging system. The operation parameters may optionally further comprise imaging parameters. The imaging parameters may also be obtained using the AI model on the basis of the input data input into the AI model.

In other words, the method allows for a coordinated operation wherein injection of contrast agent and imaging are coordinated by determining operation parameters based on sensor data. The sensor data allows to do so in accordance with the actual situation at hand, specifically, the specific patient and patient-specific imaging procedure at hand. The sensor data may, alone or together with other input data, allow for deriving technical requirements and, accordingly, operation parameters for the injection and the imaging.

As such, the above-described objective can be achieved.

Contrast medical imaging is commonly referred to as contrast enhanced medical imaging in the radiology community and technical field. Contrast (enhanced) medical imaging techniques involve medical imaging modalities, such as CT or MRI, wherein a contrast agent, also referred to as contrast medium, is injected into a subject, e.g. patient. The medical images are acquired by a medical imaging device with contrast agent being present in an anatomical structure that is in the field of view, FOV, of the imaging device at the time of image acquisition, particularly with contrast agent being present in a region of interest, ROI, of the imaging procedure. Contrast medium will generally be injected intravenously and travel to the region of interest. Image quality depends on the amount of contrast agent being in a target anatomy in the region of interest at the time of image acquisition. Image quality, in the present disclosure, particularly may refer to clinical image quality. Clinical image quality may describe the quality or suitability of an image with respect to a subsequent clinical workflow, such as diagnosis. The amount of contrast agent in the target anatomy in the region of interest depends on travel time and distance from the injection site to the region of interest. These, in turn, depends on patient anatomy and physical state, such as pulse or blood flow.

It will be understood that there can be a vast difference between patients and imaging sessions in terms of patient anatomy, such as size, weight, vascular structures, , and current physical state of the patient, such as pulse or blood flow, to name but a few.

Such differences may be used to characterize a patient, e.g., by patient parameters.

In other words, parameters that characterize the patient, e.g. the patient anatomy and/or physical state of the patient, are herein referred to as patient parameters. The patient parameters may also be seen as parameters reflecting patient characteristics. The patient parameters may be multi-modal patient parameters, e.g. obtained from different sensor types or from one or more sensors and from data storage and/or user input.

The input data being representative of patient parameters may comprise patient parameters. For example, the input data may comprise age or weight or the like. Input data being representative of patient parameters alternatively or in addition may comprise data from which such patient parameters can be derived. That is, data may be directly used as input data, i.e. without actually deriving patient parameters. One example for using data directly is data provided by the sensors. Sensors might provide data that may be processed to determine patient parameters, such as weight or size or the like, and the patient parameters may be part of the input data. Sensor data may alternatively or in addition be used as such as input data. In other words, at least some data acquired by the one or more sensors may be directly input into the AI model, or in yet other words sensor data as acquired by the one or more sensors may be used as input.

As an example, pressure data from pressure sensors may be input into the AI model. The pressure data may be representative of a patient parameter, i.e., a patient's weight. The weight might be determined and used as part of the input data or the pressure data may be used as part of the input data without determining the weight.

Thus, as seen above, the input data may comprise sensor data as acquired by the one or more sensors and/or data derived from the sensor data, particularly one or more patient parameters derived from the sensor data.

Optionally, the input data may also comprise data retrieved from storage media and/or data input by a user via a user interface.

Generating the input data may comprise assembling a set of data for use as input data. Assembling the set of data may comprise retrieving sensor data and optionally deriving data from the sensor data, particularly patient parameters, and optionally selecting among the sensor data and/or derived data a subset to be included in the input data. The assembling may further comprise retrieving data from storage media and/or receiving data input by a user, and optionally selecting, among the retrieved and/or received data, a subset to be included in the input data. The criteria for assembling may be based on the case at hand, e.g., what kind of imaging procedure is to be carried out, and/or the AI model to be used, and, for example, what input this model expects.

Generating the input data may also comprise bringing the assembled data in such a form that it can be ingested by the AI model. The form may depend on the AI model and its determination can be implemented by a skilled person without difficulty for the AI model at hand.

According to the present disclosure, at least some of the input data is obtained from sensor data acquired by one or more sensors, e.g. comprise or are derived from the sensor data. As seen above, this does not preclude that the input data also comprise data that are retrieved or received from other sources.

The AI model may be any known AI model that has been configured, e.g. trained, to ingest the input data and output the operation data. In particular, the AI model may be a machine learning model. The machine learning model may be any known machine learning model that has been trained to ingest the input data and output the operation data. Particularly, a deep learning network may be used, such as a multi modal deep learning network. The method may comprise configuring, particularly training, the AI model to ingest the input data and output the operation data.

The AI model may be a multistage model comprising of different individual models which combined output the operation parameters. In particular, some of the models of the multistage model may be applied in sequence.

According to the present disclosure, the method comprises obtaining, making use of the AI model, operation parameters for operation of a contrast medical imaging system, the operation parameters comprising contrast injection parameters, and optionally comprising imaging parameters. The contrast medical imaging system may hereinbelow also be referred to as medical imaging system for the sake of brevity.

In other words, the output of the AI model obtained when inputting the above-described input data may comprise operation parameters for operation of the contrast medical imaging system.

The contrast medical imaging system, as will be described in detail below, may comprise at least an injector system for injecting the contrast agent and a medical imaging device for acquiring the medical images.

The contrast medical imaging system may comprise the sensor or sensors used for obtaining the sensor data.

Operation parameters of the contrast medical imaging system may comprise contrast injection parameters and optionally imaging parameters. The operation parameters may optionally also comprise other operation parameters, such as for the sensor(s) or other components of the contrast medical imaging system.

Contrast injection parameters, also in short referred to as injection parameters, may comprise parameters for operation of an/the injector system. Examples will be provided further below.

Imaging parameters may comprise parameters for operation of the medical imaging device. Examples will be provided further below.

According to the present disclosure, operation of the medical imaging system may take into account the patient at hand, particularly due to using the patient parameters. For example, injection of contrast agent, such as amount and/or timing, will take into account the patient at hand. Similarly, operation of the imaging device, such as radiation dose, may also take into account the patient at hand. Moreover, coordinated operation between injection and imaging can be carried out based on the respective operation parameters.

It is noted that, for example, the input data may comprise data including and/or indicating operation parameters for the imaging device. In this case, the output of the AI model may comprise injection parameters and optionally modified or additional operation parameters for the imaging device. Alternatively or in addition, the input data may comprise data indicating injection parameters and the output of the AI model may comprise imaging parameters and optionally modified or additional injection parameters. By already taking into account some operation parameters as input data for the AI method, this may improve the results, particularly in terms of coordination. For example, an input might specify that the imaging involves bolus tracking. This may yield better output regarding injection and optionally imaging parameters than if this was not taken into account.

According to the present disclosure the one or more sensors may comprise at least one of a camera and/or surface scanner, a weight sensor, a medical imaging device used for the medical imaging, such as CT scanner or MRI scanner. Optionally, the one or more sensors may comprise a pulse sensor and/or a blood pressure sensor.

According to the present disclosure, the sensor data may comprise image data obtained by an optical scanner and/or image data obtained by a camera. Alternatively or in addition, the sensor data may comprise pressure data from a pressure sensor. Alternatively or in addition, the sensor data may comprise medical image data from the medical imaging device, such as 2D or 3D survey data. Optionally, the sensor data may comprise pulse and/or blood pressure measurement data.

Existing medical imaging systems comprise at least the medical imaging device. Moreover, many existing medical imaging systems comprise cameras, e.g. for alignment purposes. Sensors such as weight sensors or optical sensors can also be easily incorporated in existing systems, usually at relatively low cost. Accordingly, using this type of sensors and their sensor data allows for easy retrofitting for existing systems.

According to an aspect of the present disclosure, the one or more sensors may comprise a camera and at least one of the plurality of patient parameters may be derived from image data acquired by the camera. As an example, in this case, the plurality of patient parameters may comprise at least one of patient dimensions, patient body surface area, patient heart rate, blood flow, lean body mass, weight, BMI.

The camera may be a 2D or 3D camera. The camera may, for example, provide RGB image data and/or IR image data. Image data from a camera may allow for deriving patient parameters either from the image data alone or in combination with other data, such as data obtained by other sensors, e.g. a pressure sensor which may be an indicator of weight, and/or the medical imaging device, which may provide additional image data that depicts patient anatomy not visible for a camera. Patient parameters may thus be derived from the image data alone or in combination with other sensor data. The patient parameters may be used as input for the AI model. In principle patient parameters may be derived at least also in part based on user input. The patient parameters may be derived partially or fully automatically. Suitable image processing techniques for automatically deriving patient parameters from images are known in the art. Such techniques may include segmentation, registration, edge detection, object detection, or the like. Optionally, AI-based, particularly ML-based, techniques may be employed for deriving patient parameters.

It is noted that alternatively or in addition to using patient parameters derived from the image data as part of the input data for the AI model, the image data as such may be used as part of the input data for the AI model. This has been explained above in detail.

The advantage of using cameras is that they will often already be present with medical imaging systems or are easy to add to the setup. Moreover, due to advanced image processing techniques that are available, a wealth of information can be obtained from such images, such that it may be possible to obtain several different patient parameters based on the images.

According to an aspect of the present disclosure, the one or more sensors may comprise a medical imaging device used for the medical imaging. For example, the one or more sensors may comprise a CT or MRI scanner.

At least one of the plurality of patient parameters may be derived from image data acquired by the medical imaging device, particularly from a 3D surview. For example, at the beginning of a medical imaging session or prior to the medical imaging session, a surview may be obtained, for example by acquiring medical images using the modality that will be used for the contrast medical imaging or by acquiring medical images using another medical imaging modality. Such a surview may, particularly, yield 3D image information.

This will yield information on patient anatomy not visible in image data obtained by cameras or surface scanners. For example, information concerning tissue, organs, bones, vessels, and the like can be obtained.

The patient parameters derived from image data acquired by the medical imaging device may include at least one of, in particular may be a combination of two or more of vascular characteristics, particularly length and/or diameter of vascular structures, such as vessels, cardiac size, ventricle size, particularly left ventricle size for cardiac output estimation, diameter and/or cross-sectional area of tissue and/or anatomical structures, body fat ratio and/or fat-free mass, e.g. based on tissue segmentation, anatomical landmarks, position of a bolus region of interest, ROI, distance between bolus region of interest, ROI, and target anatomy.

Patient parameters may be derived from the image data acquired by the medical imaging device alone or in combination with other sensor data. The patient parameters may be used as input for the AI model. In principle patient parameters may be derived at least also in part based on user input. The patient parameters may also be derived partially or fully automatically. Suitable image processing techniques for automatically deriving patient parameters are known in the art. Such techniques may include segmentation, registration, edge detection, object detection, or the like. Optionally, AI-based, particularly ML-based, techniques may be employed for deriving patient parameters.

It is noted that alternatively or in addition to using patient parameters derived from the image data acquired by the medical imaging device as part of the input data for the AI model, the image data as such may be used as part of the input data for the AI model. This has been explained above in detail.

Using the medical imaging device for obtaining sensor data is advantageous, particularly where medical images would have to be taken anyway in the medical imaging workflow, as this means that additional information on the patient can be gained without additional hardware and potentially even without additional data acquisition. It may be particularly advantageous when combined with image data from a camera, as this will provide external and internal views of the patient, which, together may yield more details and/or higher quality input data for the AI model.

According to the present disclosure, the one or more sensors may comprise a weight sensor and the at least one of the plurality of patient parameters may be a weight of the patient and/or derived from the weight of the patient, such as BMI or body fat ratio.

Patient parameters may be derived from the weight data acquired by the medical imaging device alone or in combination with other sensor data, such as image data from a camera and/or a medical imaging device. The patient parameters may be used as input for the AI model. In principle patient parameters may be derived at least also in part based on user input. The patient parameters may be derived partially or fully automatically. For example, calculations or models may allow deriving patient parameters from the weight data. An example would be BMI calculation, which uses weight and height of a patient as input for calculating a BMI value. The weight may be the weight data output by the weight sensor. The height may be input by a user or automatically determined from image data, e.g. as explained above. Optionally, AI -based, particularly ML-based, techniques may be employed for deriving patient parameters.

It is noted that alternatively or in addition to using patient parameters derived from weight sensors as input for the AI model, the weight data acquired by the weight sensors as such may be used as input for the AI model.

Using weight sensors can be advantageous, as self-reported information may not be up-to-date or wrong for other reasons. Significant misreporting of weight might even endanger the patient, and in any case will lead to lower quality results.

According to the present disclosure, the input data may additionally be representative of information retrieved from a storage and/or input by a user. For example, a user may input the information via a user interface at the time of the imaging procedure or at an earlier time.

The information retrieved from storage and/or input by a user may include patient information, such as clinical indication, co-morbidities, weight, BMI. The information may comprise patient parameters derived previously from sensor data and stored in the storage and/or may comprise previously obtained sensor data, e.g. from previous medical exams. The information may also comprise information entered by a user, such as co-morbidities or clinical indication. For some information, e.g. weight or BMI, the information may be based on sensor data or a user input. For example, a user input may supplement missing information in the sensor data and/or sensor data may supplement missing information in the user input. Co-morbidities may, for example, comprise renal function or allergies, such as allergies to contrast media. For example, patient information may be retrieved from electronic medical records, EMR, of a patient.

The information retrieved from storage and/or input by a user may include information concerning the medical imaging procedure, e.g. imaging parameters, such as target anatomy and/or target scanner parameters, like kVp. Such information may, for example, be stored in a storage exam card.

According to the present disclosure, the contrast injection parameters may include at least one of: amount of contrast agent, flow rate of contrast agent, timing of injection, needle selection, optionally saline flush parameters, such as saline flush flow rate and/or saline flush amount.

A set of injection parameters, for example the set of injection parameters required for the medical imaging workflow, may be referred to herein as injection protocol.

The amount of contrast agent may be given as a volume. The flow rate may be given as volume per time, for example cubic centimeters per second (cc/sec).

The needle selection may, for example, indicate a diameter of a needle to be used and/or may identify a specific needle type, e.g. using any type of identification, such as a name, number, code, or the like.

The needle selection may comprise a suggested needle selection and/or a current needle selection. That is, the AI model may output which needle selection would be suggested to be used for the injection. Alternatively or in addition, if the sensor data allow for deriving a current needle selection, the AI model may output which needle is currently selected. As an example, a camera image may depict the currently selected needle and this camera image may be ingested by the AI model, which may then indicate the currently selected needle.

Optionally, the AI model may output an indication that a current needle selection corresponds to a suggested needle selection and/or that the current needle selection does not match the suggested needle selection. The latter may cause an alert to be issued. In other words, a current selection or choice of a needle may be validated using the AI model. Using the AI model to validate the needle selection allows to select the adequate needle for the patient at hand, e.g. taking into account patient parameters like vein size) and/or for the medical imaging examination procedure at hand, e.g. if the input data also comprises information on the examination. This may allow for taking into account examination requirements, such as flow rates required for the examination.

Alternatively or in addition, the AI model may be trained to output other injection parameters, such as an admissible range of flow rates, taking into account a currently selected needle. The currently selected needle may be specified in the input data for the AI model, for example determined automatically from sensor data, e.g. by image processing of images acquired by a camera, and/or input by a user. Alternatively or in addition, the AI model may be trained to take sensor data, e.g. images depicting a needle, as part of the input data and outputting operating parameters that differ for different needle selections.

Similarly, alternatively or in addition, the AI model may be trained to output operation parameters, particularly injection parameters, taking into account a needle position relative to the patient anatomy. The needle position may be specified in the input data for the AI model, for example determined automatically from sensor data, e.g. by image processing of images acquired by a camera, and/or input by a user. Alternatively or in addition, the AI model may be trained to take sensor data, e.g. images depicting a needle and at least part of the patient anatomy where the needle is positioned, as part of the input data and outputting operating parameters that differ for different needle positions.

According to the present disclosure, the timing of injection may be an absolute timing or a timing relative to other steps of the medical imaging procedure, particularly relative to medical image acquisition. Thus, timing of injection is an output that is particularly important for coordinated operation of the injector system and the imaging device. That is, the AI model may output timing of injection and timing of medical image acquisition, potentially as relative timing. Thereby, injection of contrast agent and acquisition of medical images can be coordinated efficiently. This improves image quality, but it also allows reducing the amount contrast agent, as otherwise the contrast agent might be injected for a longer period of time than is necessary for the imaging procedure.

According to the present disclosure, the imaging parameters comprise at least one of: scanner settings, spectral imaging reconstruction parameters, bolus region of interest, ROI, placement, timing of scan, particularly of a locator scan.

According to the present disclosure, the term scanner settings may be understood as settings of the medical imaging device. as an example, scanner settings may include dose, kVp, scanner pitch, table speed, or the like.

Timing of scan may be an absolute timing of one or more image acquisitions. The timing may be an absolute timing or may be a relative timing, particularly a relative timing with respect to timing of contrast agent injection. As explained above, this allows for coordinated operation of imaging and injection and may improve image quality and reduce contrast agent amount.

One specific example for parameters relating to timing of scan is in the context of bolus tracking, e.g., where the region of interest tracks the bolus of the contrast agent as it arrives in the target anatomical region. The present disclosure, taking into account patient parameters, allows for improved timing of image acquisition in this context, as the movement of the bolus depends on patient parameters, such as anatomy and/or blood flow.

According to the present disclosure, the timing of the scan may be determined relative to the time of injection and may be determined based on a bolus travel time to a target anatomy, e.g. a target vasculature, derived from heart rate and/or cardiac output and/or speed of blood-flow together with vasculature parameters, particularly bolus travel distance to the target anatomy, e.g. target vasculature. Particularly, the present disclosure may provide patient-specific bolus timing calculations based on the sensor data and optionally updated scan parameters.

Optionally, the method of the present disclosure may comprise issuing an alert when actual timing of the image acquisition is not in synchronization with a bolus travelling through the patient.

According to the present disclosure, the AI model may, for example, comprise a deep learning model, particularly a multi-modal deep learning model. Such AI models yield particularly good results with the input parameters of the present disclosure.

According to the present disclosure, the AI model may be trained based on expert annotation of medical image quality. That is, an AI model may be used to predict injection and optionally imaging parameters, and images resulting from such parameters may be assessed by experts and annotated according to the assessment. Based thereon, the AI model may be trained to output parameters yielding images of a given quality.

Alternatively or in addition, the AI model may be trained based on automatically assessed medical image quality, for example assessed using another AI, particularly ML, model. Commonly known AI models for assessing image quality may be applied for this assessment, for example.

According to the present disclosure, the method may comprise that the injection parameters are provided to an injector system and, optionally after user confirmation, are used to set operation settings of the injector system for the injection of contrast agent for the contrast medical imaging.

For example, a user interface may be provided where the injection parameters output by the AI model are presented to the user, for example auto-filled, and the user may confirm via an input device to proceed with the presented injection parameters.

The method may comprise, in case the user does not confirm the injection parameters, a modification thereof, automatically and/or by the user via the input device. The modification may be carried out, e.g. repeated, until the user confirms the modified parameters. Then, the method may proceed with the confirmed injection parameters.

The user confirmation may be carried out prior to or after providing the injection parameters to the injector system, e.g. depending on whether the user interface is provided at the injector system or separate from the injector system.

The method of the present disclosure may comprise the medical imaging device and a/the injector system coordinating operation, based on the injection parameters and/or the imaging parameters, particularly coordinating start of the injection and the time of scan based on the injection parameters and/or the imaging parameters,

For example, coordinated operation may entail taking into account a travel time of the contrast agent from the position where it is injected to the region of interest. Imaging may be carried out at a time when, based on the presumed travel time, the ideal amount of contrast agent is in the region of interest. Alternatively or in addition, where a bolus of contrast agent is followed as part of the imaging procedure, the following may be controlled based on the expected movement of the bolus through the patient.

The present disclosure also provides a contrast medical imaging method comprising the method as described above and/or specified in the claims, the contrast medical imaging method comprising acquiring medical imaging data based on the operation parameters, particularly the injection parameters and/or imaging parameters obtained by the method as described above and/or specified in the claims.

The present disclosure also provides a medical imaging system configured to carry out the method of the present disclosure, particularly as outlined above and/or as claimed.

The medical imaging system may be configured to generate, for example by a processing device, input data representative of a plurality of patient parameters, wherein at least some of the input data is obtained from sensor data acquired by one or more sensors. The medical imaging system is further configured to input, for example by the processing device, the input data into an artificial intelligence, AI, model, particularly a machine learning, ML, model, and to obtain, using the AI model, operation parameters for operation of a contrast medical imaging system, for example by the processing device, the operation parameters comprising contrast injection parameters, and optionally comprising imaging parameters.

The medical imaging system may comprise the processing device described above. The medical imaging system may comprise a/the medical imaging device configured to acquire medical image data, e.g., in accordance with the imaging parameters. The medical imaging device may comprise a CT scanner or MRI scanner. The medical imaging system may comprise the one or more sensors. The sensors may comprise at least one of a camera and/or surface scanner, a weight sensor, a/the medical imaging device used for the medical imaging, such as CT scanner or MRI scanner.

The medical imaging system may comprise an injector system for injection of contrast agent, e.g. in accordance with the injection parameters.

The present disclosure also provides a computer program product comprising computer-readable instructions which, when executed by a computer, cause the computer to carry out and/or control the method of the present disclosure, particularly as outlined above and as claimed.

The present disclosure also provides a computer-readable medium having stored thereon computer-readable instructions which, when executed by a computer, cause the computer to carry out and/or control the method of the present disclosure, particularly as outlined above and as claimed.

The features and advantages outlined in the context of the method similarly apply to the system, computer program product, and computer-readable medium.

### BRIEF DESCRIPTION OF THE DRAWINGS

Fig 1 illustrates a method for use in contrast medical imaging according to the present disclosure,
Fig. 2 illustrates a schematic, not to scale view of a medical imaging system according to the present disclosure.
Fig. 3 illustrates an exemplary workflow according to the present disclosure, particularly exemplary potential inputs and outputs of the AI model and exemplary communication between components of the system according to the present disclosure.

### DETAILED DESCRIPTION OF EMBODIMENTS

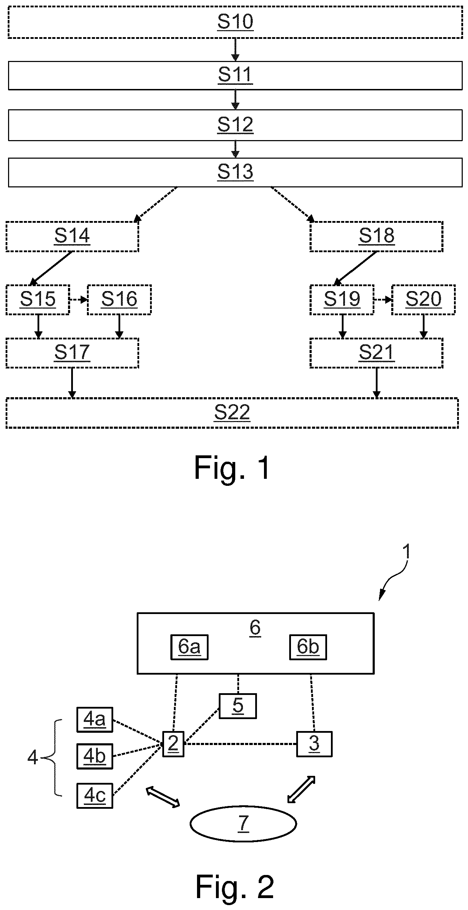

Fig 1 illustrates a method for use in contrast medical imaging according to the present disclosure.

The method comprises, in step S 11, generating input data representative of a plurality of patient parameters, wherein at least some of the input data is obtained from sensor data acquired by one or more sensors. In particular, at least some of the patient parameters may comprise or be derived from the sensor data.

As explained above, the patient parameters may be determined from sensor data and the input data may comprise the patient parameters. The method may comprise the determination of patient parameters, e.g., based on image processing techniques in case the sensor data comprises image data. Alternatively or in addition, the input data may comprise the sensor data or pre-processed sensor data, where pre-processing may comprise conversion, compression, and/or enhancement of sensor data, for example. Moreover, optionally, the input data may comprise data retrieved from storage and/or data input by a user, or data derived therefrom. This may include patient information, such as clinical indication, co-morbidities, weight, BMI, and/or information concerning the medical imaging procedure, such as target anatomy and/or target scanner parameters. The method may comprise retrieving the data from storage and/or receiving the data input by a user and optionally processing said data to obtain data derived therefrom. The generating may comprise assembling data to be included in the input data and optionally bringing the assembled data into a form that can be used as input data for the AI model used in step S 12 described below.

The one or more sensors may comprise at least one of a camera and/or surface scanner, a weight sensor, a medical imaging device used for the medical imaging, such as CT scanner or MRI scanner. For example, the one or more sensors may comprise a camera and at least one of the plurality of patient parameters may be derived from image data acquired by the camera and comprises at least one of patient dimensions, patient body surface area, patient heart rate, blood flow, lean body mass, weight, BMI. The one or more sensors may comprise the medical imaging device used for the medical imaging and at least one of the plurality of patient parameters may be derived from image data acquired by the medical imaging device, particularly from a 3D surview. Such patient parameters may include at least one of, in particular may be a combination of, two or more of vascular characteristics, particularly length and/or diameter of vascular structures, such as vessels, cardiac size, ventricle size, particularly left ventricle size for cardiac output estimation, diameter and/or cross-sectional area of tissue and/or anatomical structures, body fat ratio and/or fat-free mass e.g. based on tissue segmentation, anatomical landmarks, position of a bolus region of interest, ROI, distance between bolus region of interest, ROI, and target anatomy. The one or more may sensors comprise a weight sensor and the at least one of the plurality of patient parameters may be a weight of the patient and/or derived from the weight of the patient, such as BMI or body fat ratio.

The method comprises, in step S12, inputting the input data into an AI model, particularly an ML, model. The AI model may comprise a deep learning model, particularly a multi-modal deep learning model. The latter can cope particularly well with the type of input data generated in step S11.

The method comprises, in step S13, obtaining, using the AI model, operation parameters for operation of a contrast medical imaging system, the operation parameters comprising contrast injection parameters and optionally comprising imaging parameters. Particularly, the operation parameters obtained using the AI model may comprise both, injection parameters and imaging parameters.

The contrast injection parameters may include at least one of: amount of contrast agent, flow rate of contrast agent, timing of injection, needle selection, optionally saline flush parameters, such as saline flush flow rate and/or saline flush amount.

The imaging parameters may comprise at least one of: scanner settings, spectral imaging reconstruction parameters, bolus region of interest, ROI, placement, timing of scan, particularly of a locator scan.

The timing of injection and timing of scan may be output, particularly a relative timing between injection and scan. This allows for coordinated operation of the injection and the medical imaging.

As an example timing of the scan may determined relative to the time of injection and determined based on a bolus travel time to a target vasculature derived from heart rate and/or cardiac output and/or speed of blood-flow together with vasculature parameters, particularly bolus travel distance to the target vasculature.

The method may comprise training the AI model in optional step S10. The AI model may be trained based on expert annotation of medical image quality. Alternatively or in addition, the medical image quality may be automatically assessed, for example by means of another AI model, e.g. ML model.

The method may comprise, in optional step S14, providing the injection parameters to an injector system. In an optional step S15, the injection parameters may be presented to a user for confirmation, for example via a user interface. If the user confirms the injection parameters, in step S17, the injection parameters are used to set operation settings of the injector system for the injection of contrast agent for the medical imaging. If the user does not confirm, the injection parameters may be modified in optional step S16 prior to being used to set operation settings of the injector system for the injection of contrast agent for the medical imaging in step S17. As an example, the user may modify at least some of the injection parameters via the user interface or modification of the injection parameters may be automatically performed, optionally repeatedly until a user confirms the injection parameters.

The user confirmation, e.g. steps S15 to S16, may be carried out prior to or after providing the injection parameters to the injector system in step S14. This may depend on where calculations are performed and where the user interface is provided, for example.

In an optional step S18, the imaging parameters may be presented to a user for confirmation, for example via a user interface. If the user confirms the imaging parameters, in step S19, the imaging parameters are used to set operation settings of the imaging device for carrying out the medical imaging in step S21. If the user does not confirm, the imaging parameters may be modified in optional step S20 prior to being used to set operation settings of the imaging device in step S21. As an example, the user may modify at least some of the imaging parameters via the user input, or modification of the injection parameters may be automatically performed, optionally repeatedly until a user confirms the injection parameters.

In optional step S22, the medical imaging device and the injector system may coordinate operation based on the injection parameters and/or imaging parameters. As an example, the start of the injection and the time of the scan and/or the movement of the scanner may be coordinated. For example, to that end, the method may comprise communication between the injector system and the medical imaging device or communication between a third device and the injector system and medical imaging device, the communication transmitting data allowing for coordinated operation, e.g. trigger signals or the like.

A method comprising the preceding steps including medical imaging may be considered a medical imaging method according to the present disclosure.

The above are only some examples and steps may be omitted or modified and/or steps may be added.

Fig. 2 is a schematic illustration of a medical imaging system 1 according to the present disclosure. The medical imaging system is configured to carry out a method according to the present disclosure, for example as explained in the context of Fig. 1. Any features described in the context of the method may similarly apply to the system and vice versa.

The medical imaging system may comprise a processing device 2 configured to carry out any data processing steps of the present disclosure. The data processing device may be one computing component or comprise a distributed system wherein different computing components are connected via data connections. The method of the present disclosure may be carried out in a centralized manner or in a distributed manner, i.e., distributed over the different computing components of a/the distributed system. At least part of the method of the present disclosure may be carried out in a cloud system, for example.

The medical imaging system may comprise a/the medical imaging device 3 configured to acquire image data, e.g., in accordance with the imaging parameters. The medical imaging device may comprise a CT scanner or MRI scanner.

The medical imaging system may comprise the one or more sensors 4. The sensors may comprise at least one of a camera 4a and/or surface scanner 4b, a weight sensor 4c. The sensors may optionally comprise the medical imaging device 3.

The medical imaging system may comprise an injector system 5 for injection of contrast agent, e.g. in accordance with the injection parameters.

The medical imaging system may comprise a user interface 6 configured to allow for a user to provide user inputs to be processed by the computing system. As an example, the user interface may comprise a display portion 6a and one or more input devices 6b. The display portion itself may also be an input device, e.g., a touch screen.

A patient 7, not part of the system, is shown for the sake of illustration.

The medical imaging device may comprise data connections, indicated by dashed lines, which connect some or more of the components of the medical imaging system.

For example, a data connection between the injector system and the medical imaging system may allow for communication between the injector system and the medical imaging device for coordinating operation of the medical imaging device and the injector system. Data connections between a third device and the injector system and medical imaging device may allow for communication between the third device and the injector system and medical imaging device, the communication transmitting data for coordinating operation of the medical imaging device and the injector system.

The present disclosure also provides a computer program product comprising computer-readable instructions which, when executed by a computer, cause the computer to carry out and/or control the method of the present disclosure, for example as outlined in the context of Fig. 1.

The present disclosure also provides a computer readable medium having stored thereon computer-readable instructions which, when executed by a computer, cause the computer to carry out and/or control the method of the present disclosure, for example as outlined in the context of Fig 1.

In the following, more aspects and advantages of the method and system of the present disclosure will be provided.

In practice, a significant number of medical imaging examinations, such as CT, are conducted with intravenous injection of a contrast agent, also referred to as contrast medium. For example, iodinated contrast media can be used to enhance the image contrast between a lesion and the normal surrounding tissue. Depending on the target of the examination, the timing between intravenous injection of the contrast medium and the scan may be selected differently, for example:
Early arterial phase (15-20 sec after contrast injection): For the detection of arterial bleeding, with arteries enhanced and organ parenchyma unenhanced.

Late arterial phase (35-40 sec after contrast injection): for the detection of hypervascular liver lesions.

Portal venous phase (70-80 sec after contrast injection): Detection of hypervascular liver lesions

Delayed phase (6-10 min after contrast injection): Fibrotic metastases

In addition to the correct timing, the scan quality will also depend on the total amount of contrast agent injected. Most institutions generally use the same amount of contrast agent for all exams, or at best adapt roughly to the patient's weight from a patient file.

Another parameter presently to be set by the operator is the injection rate, e.g., the amount of contrast agent that is injected per second.

It is, for various reasons, advantageous to be able to reduce contrast medium used for imaging. Particularly, this is better for the patient. It can also help mitigating effects of disruptions of global supply chains or errors in stocking of contrast media resulting shortage of contrast media, which might otherwise severely disrupt the CT imaging workflows in hospitals and health systems.

Correct setting of injection parameters and contrast timing is perceived as a difficulty in the CT image acquisition workflow. Multiple stakeholders in the imaging workflow can be affected by wrong selection of such parameters, including technologists, radiologists and patients themselves, and the resulting problems could range from suboptimal image quality to a missed diagnosis.

Correct selection of contrast injection parameters can be difficult, which can lead to suboptimal parameter selection and poor image quality. Following standardized protocols for parameter selection, such as selection of amount and timing of contrast agent injection, can lead to an overuse of contrast agent.

The present disclosure has at least some of the following effects and advantages.

The required amount of contrast agent may depend on a variety of factors, such as the fat-free mass or the body surface area of the patient. For example, fat-free mass is an appropriate body size parameter for correlation with liver parenchymal enhancement.

The contrast uptake behavior can also depend significantly on patient parameters, such as the cardiac output. Behaviors such as the so-called Valsalva maneuver can impact the uptake of contrast in pulmonary embolism exams.

These parameters are at present not taken into account, as there is little or no personalization of contrast injection protocols, e.g. by adjusting for the self-reported weight of the patient.

The above often results in overdose to the patient and contributes to a sub-optimal use of the contrast medium and potentially introducing shortage issues.

Another issue with present methods is that medical imaging device, e.g. CT scanner, and injector system operate independently of each other. The lack of coordination leads to an inefficient operation of both systems. Moreover, the present systems lack mutual communication, which causes a need to go back-and-forth between the respective interfaces in order to establish a workflow. In extreme cases, continued contrast agent injection has been observed even after the scan acquisition was completed leading to larger amounts of contrast agent being injected than needed.

This decoupled approach regarding contrast injection and medical image acquisition combined with the lack of options for retrospective analysis of image quality issues and their possible relation to contrast injection parameters prevents effective improvement actions.

The present invention leverages patient parameters, at least some of which are determined by sensors for the imaging session at hand and furthermore leverages modelling to determine, from the sensor parameters, operation parameters for the system, such as injection parameters and/or medical imaging parameters.

Patient parameters may be obtained by or based on a, particularly multi-modal, patient parameter measurement system. The system may be configured to carry out an automatic estimation of relevant patient parameters, for example using multiple sensors, such as imaging devices. A camera setup may be equipped, for example for analyzing the patients size, body surface, heart rate etc., and/or a 2D/3D surview image of the medical imaging device may be obtained for deriving patient characteristics such as length and diameter of the relevant vascular structures, and/or pressure sensors may be provided for determining weight of a patient and patient parameters that take weight into account, such as BMI or body fat ratio.

The modelling used for obtaining operation parameters, particularly injection parameters and/or medical imaging parameters, may be an AI based system. For example, an AI based parameter definition may be carried out and may yield, as an output, a recommendation of scan parameters and/or contrast injection parameters using the input provided by the patient parameter measurement system.

The invention allows to improve the overall workflow for the acquisition of contrasted medical images, such as CT scans or MRI. The various steps along the workflow are supported by AI based analysis of the sensor data, for example including one or more of overhead camera, in-bore camera, and/or 2D/3D surview CT image and/or pressure data. Several of these sensors may already be incorporated in some existing imaging systems, such that the method can be easily onboarded.

In the following, an exemplary patient parameter measurement methods and systems according to the present disclosure are explained in detail.

In current clinical practice the contrast injection parameters either follow fixed presets or are personalized only in a rudimentary manner, e.g., based on a reported patient weight.

Exemplary injection parameters that may be adapted include: amount of contrast agent to be injected, flow rate (cc / sec), and/or follow-up with saline flush (flow rate, amount).

The present disclosure entails that patient parameters are derived from sensor data, for example:
Patient size and/or heart rate, for example determined using a camera
Patient weight, for example determined using a pressure sensor
Using the medical imaging modality for obtaining a 3D surview or localizer images. The surview image data or localizer image data may be analyzed, for example for determining body fat composition. For example, surview image data may be analyzed to obtain the patient size, e.g., diameter and cross-sectional area, over the extent of the surview FOV. Combined with patient size this allows to estimate e.g., the patient's fat-free mass.

The parameters may be measured automatically, for example in response to determining that required patient parameters are missing from an initial input data set, such as EMR patient information and/or Exam Card.

By automatically measuring the relevant parameters, the imaging and/or injection parameters, such as proposed contrast injection protocol, can be derived without intervention of the operator. The protocol may optionally be presented to the operator for approval, e.g. via a display portion of a user interface.

For example all available information from the EMR (patient information) and the Exam card (scanner settings) may be ingested by the system to automatically conduct the parameter computation. Missing patient parameters may be derived from the sensor data, particularly automatically obtained sensor data.

The sensor, more specifically a camera and the medical imaging modality and their output, i.e., camera images and/or 2D/3D surviews or localizer images, may optionally be used for an estimation of contrast injection settings such as contrast timing and needle selection in the following manner.

### Contrast timing

There are standard contrast timing ranges, e.g. ranges for a time between injection and start of the scan, for different applications.

For some scans the timing can be particularly critical. Especially for angiography scans that target the contrast bolus while travelling through the arterial system, the correct timing of the scan is critical. The timing can be improved by tracking the contrast bolus. To that end, presently, a ROI placed in the aorta is continuously monitored, which may be referred to as locator scan. The start of the scan is automatically triggered after a pre-defined time interval starting when the contrast bolus reaches the ROI.

Analyzing the vasculature, particularly distance from Bolus tracking ROI to target vasculature, the heart rate, the cardiac output, and the speed of blood-flow, the bolus travel time from the tracking ROI to the target vasculature can be estimated, particularly in a personalized manner for each patient and imaging session.

### Needle selection

Injection needles of different sizes are color coded in clinical practice. Based on camera image, e.g. RGB image, which may be captured by one of the sensors, the selected needle can be identified. The choice of needle can optionally subsequently be validated taking into account patient parameters as well as contrast flow rates required for the planned examination. It may be determined, e.g., in the validation step, whether an unsuitable needle, e.g. too small needle (insufficient maximum flow rate) or too large needle (patient veins are too small for proper injection), was chosen. In case it is determined that an unsuitable needle was chosen, for example, an alert may be output to the user.

In the following, an example of the AI-based determination of parameters, particularly recommendation of contrast injection, according to the present disclosure will be provided.

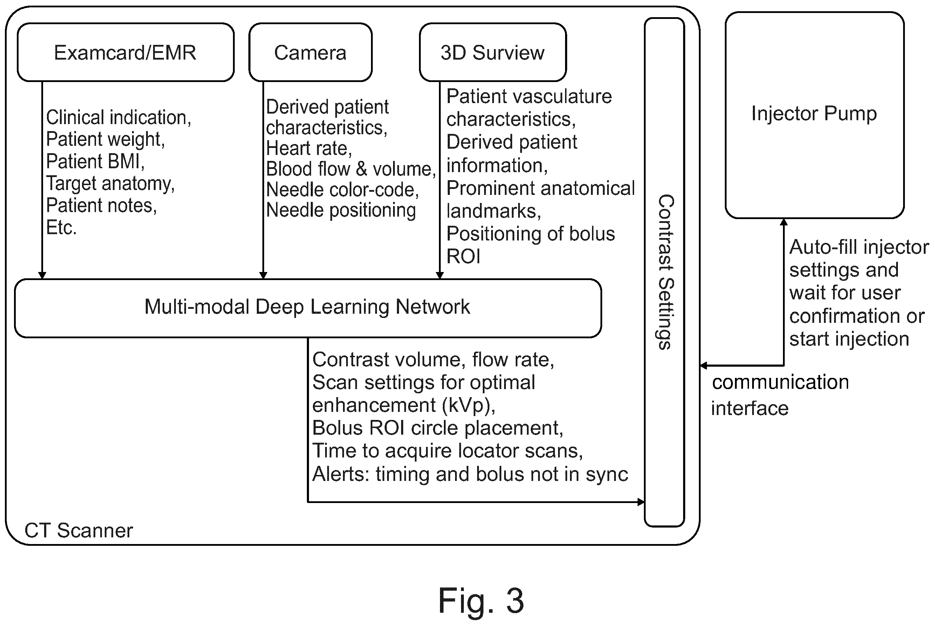

The derivation of injection and/or imaging parameters, particularly patient specific parameters and optimal scan parameters, using sensor data as input, may be carried out, as one example, using a multi-modal deep learning network, as exemplified in Fig. 3. However, other AI techniques known in the art may also be used.

Fig. 3 illustrates an exemplary workflow according to the present disclosure, particularly various potential inputs and outputs of the AI model and communication between components of the system according to the present disclosure. It is noted that the inputs and outputs are exemplary and only a subset of the shown inputs and outputs may be used and/or other inputs and outputs are possible.

Moreover, Fig. 3 illustrates a CT scanner, but other medical imaging devices are conceivable. Fig. 3 also illustrates the AI -based prediction being carried out at the CT scanner, but the prediction may be carried out on a device that is separate from the CT scanner, particularly a computing device connected to the CT scanner and other components of the system. In Fig. 3, an injection pump is shown as an example of an injector system. However, other injector systems are conceivable. Finally, Fig. 3 shows a specific communication protocol for communication between the CT scanner and the injection pump. However, these and other system components may communicate by any other suitable communication protocol.

An AI model, for example a deep learning network, may be trained for predicting from given input parameters, which injection parameters, particularly which injection protocol, and optionally which imaging parameters will result in an image quality that is determined to be sufficient (image quality may, for example, be determined as being sufficient based on rules or based on another AI technique) at the lowest possible amount of contrast agent injected.

Input parameters that may be used for the prediction may include one or more of the following parameters, but are not limited thereto:
From 2D/3D surview of the medical imaging device as one of the sensors:
   Tissue segmentation: Body fat percentage
   Cardiac size, left ventrical size (for cardiac output estimation)
   Vessel diameters
   Distance from Bolus tracking ROI to target anatomy
From a camera as one of the sensors:
   Patient size
   Patient body surface area
   Heart rate
   From stored data, such as retrieved from an EMR
   Clinical indication
   Co-morbidities, such as renal function and/or known allergic reactions to contrast media
From stored data, such as retrieved from an exam card
   Target anatomy
   Scanner parameters, e.g. kVp

The output of the AI, e.g. the deep learning network, may include one or more contrast-exam parameters, such as injection or imaging parameters. The contrast-exam parameters may comprise at least one of contrast volume, flow rate, kVp for optimal enhancement, time to acquire locator scans, bolus ROI placement etc.

The input parameters are provided to the AI model which in turn may, for example, predict appropriate contrast injection settings.

Once the injection and optionally imaging parameters have been derived, they may be auto-filled to the injector system and used immediately or only after user confirmation, e.g. via the user interface. A communication interface may be used for communication between medical imaging device, e.g. scanner, and injector system, e.g. injector pump, to coordinate workflow. For example, the communication interface may be used to start the injection, start the bolus tracking and initiate the scan at the appropriate time.

As explained above, training the AI model may involve determining image quality. Expert annotations may be used for determining the image quality of retrospective CT images can be used for training of the AI system described above Alternatively or in addition, an AI model can be trained for automatic assessment of the image quality of contrasted CT scans.

Note that the above description often mentions CT scans as an example. However, the above description also applies to contrasted MRI scans.

The present disclosure may also comprise a contrast medical imaging method comprising the method as described above and/or specified in the claims, the contrast medical imaging method comprising acquiring medical imaging data based on the operation parameters, particularly the injection parameters, and optionally imaging parameters, obtained by the method as described above and/or specified in the claims.

The present disclosure also provides a computer program product and a computer-readable medium comprising computer-readable instructions which, when executed by a computer, cause the computer to carry out and/or control the method of the present disclosure.

While the invention has been illustrated and described in detail in the drawings and foregoing description, such illustration and description are to be considered exemplary and not restrictive. The invention is not limited to the disclosed embodiments. In view of the foregoing description and drawings it will be evident to a person skilled in the art that various modifications may be made within the scope of the invention, as defined by the claims.

### LIST OF REFERENCE SIGNS:

Steps S10 to S22
Medical imaging system 1
Processing device 2
Medical imaging device 3
Sensor 4
Camera 4a
Surface scanner 4b
Weight sensor 4c
Injector system 5
User interface 6
Display portion 6a
Input device 6b
Patient 7

## Claims

1. A computer-implemented method for use in contrast medical imaging, the method comprising
generating input data (S11) representative of a plurality of patient parameters, wherein at least some of the input data is obtained from sensor data acquired by one or more sensors;
inputting the input data (S12) into an artificial intelligence, AI, model; and
obtaining (S13), using the AI model, operation parameters for operation of a contrast medical imaging system, the operation parameters comprising contrast injection parameters, and optionally comprising imaging parameters.

2. The method of claim 1, wherein the one or more sensors comprise at least one of a camera and/or surface scanner, a weight sensor, a medical imaging device used for the medical imaging, such as CT scanner or MRI scanner.

3. The method of claim 1 or 2, wherein the one or more sensors comprise a camera and at least one of the plurality of patient parameters is derived from image data acquired by the camera and comprises at least one of patient dimensions, patient body surface area, patient heart rate, blood flow, lean body mass, weight, BMI.

4. The method of any of the preceding claims, wherein the one or more sensors comprises a medical imaging device used for the medical imaging and at least one of the plurality of patient parameters is derived from image data acquired by the medical imaging device, particularly from a 3D surview, and includes at least one of, in particular is a combination of two or more of:
vascular characteristics, particularly length and/or diameter of vascular structures, such as vessels,
cardiac size,
ventricle size, particularly left ventricle size for cardiac output estimation,
diameter and/or cross-sectional area of tissue and/or anatomical structures,
body fat ratio and/or fat-free mass, e.g. based on tissue segmentation,
anatomical landmarks,
position of a bolus region of interest, ROI,
distance between bolus region of interest, ROI, and target anatomy.

5. The method of any of the preceding claims, wherein the one or more sensors comprise a weight sensor and the at least one of the plurality of patient parameters is a weight of the patient and/or derived from the weight of the patient, such as BMI or body fat ratio.

6. The method of any of the preceding claims, wherein the input data is also representative of information retrieved from a storage and/or input by a user, including patient information, such as clinical indication, co-morbidities, weight, BMI, and/or information concerning the medical imaging procedure, such as target anatomy and/or target scanner parameters.

7. The method of any of the preceding claims, wherein the contrast injection parameters include at least one of: amount of contrast agent, flow rate of contrast agent, timing of injection, needle selection, optionally saline flush parameters, such as saline flush flow rate and/or saline flush amount.

8. The method of any of the preceding claims, wherein the imaging parameters comprise at least one of: scanner settings, spectral imaging reconstruction parameters, bolus region of interest, ROI placement, timing of scan, particularly of a locator scan.

9. The method of claim 8, wherein the timing of the scan is determined relative to the time of injection and is determined based on a bolus travel time to a target anatomy, e.g. target vasculature, derived from heart rate and/or cardiac output and/or speed of blood-flow together with vasculature parameters, particularly bolus travel distance to the target anatomy, e.g. target vasculature.

10. The method of the preceding claims,
wherein the AI model is trained based on expert annotation of medical image quality and/or based on automatically assessed medical image quality, for example assessed using another AI model, and/or
wherein the AI model comprises a deep learning model, particularly a multi-modal deep learning model.

11. The method of any of the preceding claims,
wherein the injection parameters are provided to an injector system and, optionally after user confirmation, are used to set operation settings of the injector system for the injection of contrast agent for the contrast medical imaging.

12. The method of any of the preceding claims,
wherein, based on the injection parameters and/or the imaging parameters, the medical imaging device and a/the injector system coordinate operation, particularly, coordinate start of the injection and the time of scan.

13. A medical imaging system configured to carry out the method of any of the preceding claims.

14. A computer program product comprising computer-readable instructions which, when executed by a computer, cause the computer to carry out and/or control the method of any of claims 1 to 12.

15. A computer readable medium having stored thereon computer-readable instructions which, when executed by a computer, cause the computer to carry out and/or control the method of any of claims 1 to 12.
